# DEMANDE DE BREVET EUROPEEN

(11) **EP 2 789 652 A1**
(43) Date de publication de la demande: **15.10.2014**
(21) Numéro de dépôt: 13172646.5
(22) Date de dépôt: 19.06.2013
(51) Int. Cl.: C08K 5/10

(54) **Composition plastifiante**

(30) Priorité: 10.04.2013 FR 1353227
(71) Demandeur: Rhodia Opérations, 93306 Aubervilliers (FR)
(72) Inventeur: Rhers, Bouchra, 69003 LYON (FR); Martinz, Daniel, 1200 - Woluwe-Saint-Lambert (BE)
(74) Mandataire: Ridray, Annabelle

(57) **Abrégé**

L'invention concerne une composition plastifiante comportant une résine et un agent plastifiant qui comporte au moins un diester de formule générale (I) :
RO-OC-A-CO-OR (I)

dans laquelle :
- A est une chaine aliphatique linéaire ou branchée en C4 à C6, et
- R est un alkyle, un cycloalkyle ou un aryle.

L'agent plastifiant selon l'invention présente une bonne performance pour des formulations de types différents (mousse, film et pâte). Ce plastifiant gélifie rapidement, a une viscosité très basse et vieilli très bien. Le débullage efficace est l'une de ces caractéristiques phares. Les mousses préparées avec ce plastifiant ont des densités basses, une grande vitesse d'expansion et d'excellentes qualités.

## Description

L'invention concerne une composition plastifiante comprenant une résine et un agent plastifiant qui comporte au moins un diester. La présente invention concerne plus particulièrement un agent plastifiant qui comporte au moins un diester et son procédé de préparation. La présente invention concerne aussi les utilisations de la composition plastifiante.

### Etat de la technique

Les agents plastifiants majoritairement utilises sont communément appelés "General Purpose" (GP) phthalates, et représentent ainsi 88% de la consommation globale (6.7 MTon in 2011)1. Dans la famille GP phthalates, on trouve le DEHP (di-2-ethylhexyl phthalate), le DINP (di-isononylphthalate) et le DIDP (di-isodecylphthalate). La somme de ces trois phthalates représente de l'ordre 83% de la demande mondiale de plastifiants. Le DEHP à lui seul représente environ 50% de la demande mondiale.

### But de l'invention

L'invention a pour but général de fournir de nouvelles compositions plastifiantes. L'agent plastifiant selon l'invention présente une bonne performance pour des formulations de types différents (mousse, film et pâte). Ce plastifiant gélifie rapidement, a une viscosité très basse et vieilli très bien. Le débullage efficace est l'une de ces caractéristiques phares. Les mousses préparées avec ce plastifiant ont des densités basses, une grande vitesse d'expansion et d'excellentes qualités.

L'invention a notamment pour but de fournir un procédé permettant l'obtention de l'agent plastifiant.

Les inventeurs ont mis au point un procédé de préparation de composés diesters à partir de dinitriles et/ou diacides mettant en oeuvre une réaction d'hydrolyse suivie d'une étape d'estérification. Une étape de décoloration et/ ou de purification permet l'obtention d'un diester pur.

L'invention concerne encore plus particulièrement un procédé de préparation de composés diesters ramifiés tels que le diester d'acide 2-méthylglutarique (aussi appelé MGA) et le diester d'acide 2-éthylsuccinique (aussi appelé ESA).

Le diester à base de MGA seul ou en mélange avec le diester à base mélange de d'ESA possède un avenir prometteur dans le milieu des industries chimiques. Il s'agit d'un plastifiant ayant des propriétés plastifiant pouvant remplacer les plastifiants à base de phtalates et sans phtalates. Il peut être utilisé comme substitut de plastifiant tout seul ou en mélange avec d'autres plastifiants, pour des préparations/formulations de polyamides, de béton, de polyesters, de polyuréthanes, des résines chlorure de vinyls, des co-polymères ethylene-vinyl d'acétate, de caoutchouc, de mastics ou de leurs mélanges.

Le diester est obtenu soit à partir de dinitriles en passant par un intermédiaire diacide, soit à partir de diacide directement.

WO 2007/141404 propose de préparer des composés diacides par hydrolyse de composés dinitriles en présence d'un excès de composés basiques hydroxyle, le sel carboxylate obtenu étant ensuite mis à réagir avec un acide minéral pour récupérer le composé diacide.

WO 2008/062058 propose de préparer des composés diacides par hydrolyse de composés dinitriles en présence d'un excès d'acide fort minéral. WO2007/101929 propose la préparation de diester par hydrolyse de dinitriles par voie acide ou par voie basique, l'intermédiaire diacide est ensuite estérifié en présence de méthanol afin d'obtenir le diester méthylique correspondant.

La présente invention concerne une composition plastifiante comportant une résine et un agent plastifiant qui comporte au moins un diester de formule générale (I) :

RO-OC-A-CO-OR

dans laquelle :
- A peut être une chaine aliphatique linéaire ou branchée comprenant de 4 à 6 Carbones, et
- R peut être un alkyle, un cycloalkyle ou un aryle ;
en particulier R est un cycloalkyle substitué ou non, de 5 à 6 atomes de carbone ;
ou un alkyl ramifié ayant de 4 à 10 d'atome de carbone

De manière préférée R est le cyclohexyle ou le 2-éthyl-hexyle.

En particulier, le diester peut être un dérivé d'un diacide choisi parmi l'acide succinique, l'acide glutarique, l'acide adipique, l'acide ethylsuccinique ou l'acide methylglutarique.

Selon un mode d'exécution de l'invention, l'agent plastifiant comprend un mélange comprenant :
de 70 à 95% en poids de diester d'acide methylglutarique
de 5 à 30% en poids diester d'acide ethylsuccinique
de 0 à 10% en poids diester d'acide adipique

Selon un autre mode d'exécution de l'invention, l'agent plastifiant comprend un mélange comprenant :
de 95 à 100% en poids de diester d'acide méthylglutarique, et
de 0 à 5 % en poids de diester d'acide éthylsuccinique.

Selon un mode préféré de réalisation de l'invention, le diester est le di-cyclohexyle de l'acide méthylglutarique (DCH-MGA).

Selon au autre mode de réalisation de l'invention, le diester est le di-éthyl-hexyle de l'acide adipique (DCH-AA).

Selon au troisième mode de réalisation de l'invention, le diester est le di-éthyl-hexyle de l'acide méthylglutarique (DEH-MGA).

Le plastifiant de cette invention peut être préparé par estérification de diacides avec ces alcools cycliques.

### Réactions :

Lors de la réaction d'hydrolyse du composé dinitrile en présence d'acide on a la formation de diacides et de sel hydrogénosulfate d'ammonium. Ce dernier peut être valorisé sous forme de sulfates d'ammonium par sa réaction avec une autre molécule d'ammoniaque.

Schématiquement, on peut représenter la réaction d'hydrolyse d'un composé dinitrile, par exemple le 2-méthylglutaronitrile, en composé intermédiaire diacide du procédé de l'invention de la manière suivante :

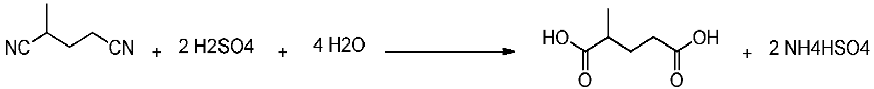

Dans le milieu réactionnel, le composé diacide est ensuite mis en réaction d'estérification avec un alcool, par exemple de cyclohexanol, pour donner un diester du procédé de l'invention de la manière suivante :

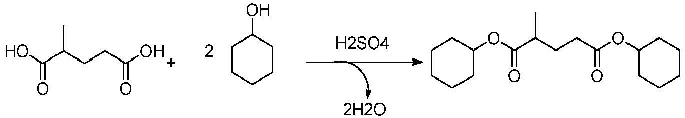

### Exemples

Les pourcentages sont donnés en poids par rapport au poids de la composition totale. Les températures sont données en degré Celsius (°C).

### Exemple 1

Procédure générale de synthèse de plastifiants par estérification.

Dans un réacteur double enveloppé de 1000ml équipé d'un système de purge en bas, d'un système d'agitation, d'une arrivée d'azote, d'un thermomètre, d'un Dean-Starck, d'une pompe pour assurer le vide et d'une ampoule équipée d'un système de refroidissement et de chauffe par laquelle sont ajoutés l'acide et d'alcool dont la stoechiométrie est 1 : 2. Le catalyseur, par exemple l'acide sulfurique est ajouté à 80°C. La réaction se déroule à 140°C avec élimination de l'eau au fur et à mesure de l'avancement de la réaction. Le stripping à l'azote favorise ainsi son élimination et sa récupération dans le Dean-starck. Une fois la quantité d'eau co-produite est totalement collectée, on monte la température au-delà de 140°C et on baisse la pression afin d'éliminer l'excès d'alcool. Le milieu réactionnel est ensuite lavé plusieurs fois avec des saumures et à la fin avec de l'eau déminéralisée. Le reste de catalyseur est neutralisé par le carbonate de sodium. Une étape de décoloration par passage sur charbon peut être faite en fonction de niveau de coloration du milieu. La phase organique contenant le diester est par la suite purifiée et décolorée par distillation. Le diester pur et incolore est ainsi obtenu avec des bons rendements.

RO-OC-A-CO-OR

**Tableau 1: sommaire des diesters préparés par réaction d'alcool et diacides.**

| Nomenclature | A | R | MM (g/mol) |
|---|---|---|---|
| Succinate de bis(2-ethylhhexyle) | -(CH₂)₂- | - 2-Ethylhexyl | 342 |
| Glutarate de bis(2-ethylhhexyle) | -(CH₂)₃- | - 2-Ethylhexyl | 356 |
| Adipate de dicyclohexyle | -(CH₂)₄- | - Cyclohexyl | 310 |
| 2-MethylGlutarate de bis(2-ethylhhexyle) | -CH₂-CH₂-CH(CH₃)- | - 2-Ethylhexyl | 370 |
| 2-MethylGlutarate de dicyclohexyle | -CH₂-CH₂-CH(CH₃)- | - Cyclohexyl | 310 |
| 2-EthylSuccinate de bis(2-ethylhhexyle) | -CH₂-CH(CH₂CH₃)- | - 2-Ethylhexyl | 370 |
| 2-EthylSuccinate de dicyclohexyle | -CH₂-CH(CH₂CH₃)- | - Cyclohexyl | 310 |
| Plast Fast = mélange de 95%MethylGlutarate de dicyclohexyle et 5% 2-EthylSuccinate de dicyclohexyle | 95% : -CH₂-CH₂-CH(CH₃)- | - Cyclohexyl | 310 |
| | 5% : -CH₂-CH(CH₂CH₃)- | | |

### Synthèse de composés à base de PVC : Plastification

Le Tableau 2 résume l'ensemble des plastifiants utilisés pour les tests avec les résines PVC sous différents formulations.

**Tableau 2: description des plastifiants**

| **Plastifiant** | **Fabricant** | **Nom chimique** | **MM (g/mol)** |
|---|---|---|---|
| DEHP | Arkema | Di (2-ethylhexyl) phthalate | 390 |
| DINP | BASF | Di-isononylphthalate | 418 |
| DINCH | BASF | Acide 1,2-cyclohexanedicarboxilique, ester diisononyl | 425 |
| DOTP | Eastman | Terephthalate de di(2-ethylhexyl) | 390 |
| Nexo E01 | Nexoleum | Methyl epoxy soyate | 327 |
| Benzoflex 2088 | Eastman | mélange d'esters benzoate (78-80% )et dipropylene glycol dibenzoate (18-20%) | 350 |
| Jayflex MB10 | Exxon | Isodecyl monobenzoate | 262 |
| Plast Fast | Solvay | Diester 2-MethylGlutarate | 310 |

Un bilan comparatif des performances des plastifiants étudiés a été réalisé pour chaque formulation. Les critères de classement de performance des plastifiants en comparaison avec le DINP sont classés comme suit :

| | | |
|---|---|---|
| 0: idem | -0,5 : légèrement moins bon | 0,5 : légèrement meilleur |
| | -1 : moins bon | 1 : meilleur |
| | -2 : mauvais | 2 : excellent |
| | -3 : très mauvais | 3 : remarquable |

### Exemple 2

Chaque plastifiant énuméré au tableau 2 permet de réaliser des formulations transparentes selon le tableau 3 ci-dessous, à l'aide d'un mélangeur type mélangeur à vitesse moyenne à une température de 23°C.

**Tableau 3: Recette de pates de PVC (couches transparentes compactes):**

| **Matières Premières** | **Quantité (phr)** |
|---|---|
| SoIVin^{®} 382NG | 100 |
| Plastifiant | 50 |
| Baerostab^{®} NT 306 (Ca/Zn) | 2.5 |

La comparaison des données obtenues pour les formulations synthétisées, à chaque fois en modifiant le plastifiant sont résumés dans le tableau 4.

Tableaux 4a à 4i : évaluations et comparaison des propriétés

**4a- Shore A : Dureté (sur plaques de 6 mm)**

| **Plastifiant** | **Cuites au Four (coupe)** | **Films Pressés** |
|---|---|---|
| DEHP | 67 | 75 |
| DINP | 72 | 78 |
| DINCH | 74,5 | 84 |
| DOTP | 71 | 76 |
| Nexo E01 | 66 | 70 |
| Plast Fast | 65 | 68 |
| MB10/DINP | 68 | 71 |
| 2088/DINP | 71 | 73 |

**4 b- viscosité à faible gradient de vitesse (Eta = 1.4 s⁻¹)**

| **Plastifiant** | **t 0** | **t0 + 24h** |
|---|---|---|
| DEHP | 4,4 | 9,6 |
| DINP | 3,6 | 15,7 |
| DINCH | 2,0 | 3,4 |
| DOTP | 3,4 | 3,4 |
| Nexo E01 | 2,4 | 4,0 |
| Plast Fast | 2,7 | 5,9 |
| MB10/DINP | 1,6 | 36,0 |
| 2088/DINP | 14,4 | 13,4 |

**4c- vitesse de gélification relative (Rhéomètre ARESs, type plat-plat, 1 rad/s, températures de 23 à 100°C, montée de 3°C/min)**

| **Plastifiants** | **Gélification** |
|---|---|
| DEHP | 0,75 |
| DINP | 0 |
| DINCH | -1,5 |
| DOTP | -0,75 |
| Nexo E01 | 1,25 |
| Plast Fast | 2,5 |
| MB10 | 1 |
| 2088 | 2 |

**4d- Perte de poids (%) à 100°C - étuve ventilée**

| **Plastifiant** | **Après 4 jours** | **Après 7 jours** |
|---|---|---|
| DEHP | 9,85 | 19,4 |
| DINP | 2,3 | 3,8 |
| DINCH | 6,3 | 10,2 |
| DOTP | 3,7 | 7,55 |
| Nexo E01 | 12,4 | 15,8 |
| Plast Fast | 20,7 | 24,6 |
| MB10/DINP | 16,4 | 17,6 |
| 2088/DINP | 4,7 | 6,9 |

**4e- Couleur, brillance et transparence des Films PVC (0.7 mm, 190°C, 2 min)**

| **Plastifiant** | **Indice de Jaune** | **Brillance** | **Transparence** |
|---|---|---|---|
| DEHP | 8,4 | 29,3 | 83,7 |
| DINP | 8,7 | 30,6 | 86,5 |
| DINCH | 8,1 | 35,5 | 85,35 |
| DOTP | 7,75 | 30,05 | 81,7 |
| Nexo E01 | 17,8 | 22,6 | 74,1 |
| Plast Fast | 11,4 | 29,1 | 87,9 |
| MB10/DINP | 8,0 | 23,2 | 82,6 |
| 2088/DINP | 9,3 | 25,8 | 82,4 |

**4f- Débullage**

| **Plastifiant** | **Hauteur colonne (mL)** | **Temps (s)** |
|---|---|---|
| DEHP | 98 | 49,5 |
| DINP | 100,5 | 53 |
| DINCH | 100 | 60,5 |
| DOTP | 93 | 29 |
| Nexo E01 | 29 | 23 |
| Plast Fast | 94 | 37 |
| MB10/DINP | 83 | 22 |
| 2088/DINP | 100 | 100 |

**4g- Stabilité thermique (DHC à 180°C et Metrastat à 197°C)**

| **Plastifiant** | **DHC (min)** | **Metrastat (min)** |
|---|---|---|
| DEHP | 25 | 10 |
| DINP | 24,2 | 15 |
| DINCH | 28,3 | 11,6 |
| DOTP | 25,8 | 15,5 |
| Nexo E01 | 152 | 27 |
| Plast Fast | 27 | 19,15 |
| MB10/DINP | 25,8 | 19,3 |
| 2088/DINP | 30,6 | 18,5 |

**4h- Compatibilité plastifiant avec PVC ASTM D3291-92**

| **Plastifiant** | **0** | **1 Jour** | **1 semaine** | **2 semaines** | **4 semaines** | **8 semaines** |
|---|---|---|---|---|---|---|
| DINP | 0 | 0 | 0 | 0 | 0 | 0 |
| Plast Fast | 0 | 0 | 0 | 0 | 0 | 0 |

**4i- Migration du film PVC (0.7 mm, 190°C, 2 min)**

| **Plastifiant** | **Migration** | **Date de fabrication du film** | **Remarques** |
|---|---|---|---|
| DEHP | 0 | sept-09 | échantillon sec |
| DINP | 0 | sept-09 | échantillon sec |
| DINCH | 0 | sept-09 | échantillon sec |
| DOTP | 0 | avr-10 | échantillon sec |
| Nexo E01 | -0,5 | avr-10 | présence d'exsudation sur le papier |
| Plast Fast | 0 | nov-12 | échantillon sec |
| MB10/DINP | 0 | Aout-10 | échantillon sec |
| 2088/DINP | 0 | Aout-10 | échantillon sec |

Les meilleures performances qui ressortent pour le plastifiant « Plast Fast » sont la gélification et le débullage. La figure 1 illustre ce phénomène et la figure 2 présente les photos prises pour le débullage. A la figure 1, il faut comprendre que les photos de la de la 2^{e} colonne sont prise après 1 minute de reprise du mélange (c'est-à-dire réincorporation d'air). Idem pour les photos de la 3^{e} colonne, après 5 minutes.

Le tableau 5 récapitule le classement des plastifiants selon les notes attribuées.

**Tableau 5. Récapitulatif des propriétés obtenues avec les plastifiants dans les formulations de pates transparentes de PVC**

| **Plastifiant** | **Viscosi té** | **Vieillis.** | **Gélif.** | **Efficaci té** | **Débul.** | **Stabilité thermiqu e** | **Couleur** | **Transp.** | **Brillan ce** | **Perte de masse** | **Migrati on** | **Score** |
|---|---|---|---|---|---|---|---|---|---|---|---|---|
| DEHP | 0 | 0,5 | 0,75 | 1 | 0 | -0,125 | 0 | 0 | 0 | -2 | 0 | 0,125 |
| DINP | 0 | 0 | 0 | 0 | 0 | 0 | 0 | 0 | 0 | 0 | 0 | 0 |
| DINCH | 0,5 | 1,25 | -1,5 | -0,75 | 0 | 0 | 0 | 0 | 0,75 | -1 | 0 | -0,75 |
| DOTP | 0 | 1,25 | -0,75 | 0,25 | 0 | 0 | 0 | -0,5 | 0 | -0,75 | 0 | -0,5 |
| Nexo E01 | 0 | 1 | 1,25 | 1,375 | 1 | 3 | -1,5 | -0,75 | -0,5 | -1,5 | -0,5 | 2,875 |
| Plast Fast | 0,25 | 0,75 | 2,5 | 1,5 | 0,75 | 0,25 | -0,5 | 0 | 0 | -2,75 | 0 | 2,75 |
| MB10/DINP | 0,75 | -0,5 | 1 | 1,125 | -0,5 | 0,125 | 0 | -0,25 | -0,5 | -1,75 | 0 | -0,5 |
| 2088/DINP | -2 | 0 | 2 | 0,7 | -1,5 | 0,25 | -0,25 | -0,25 | -0,25 | -0,5 | 0 | -1,81 |

### Exemple 3

Des formulations mousses correspondant au tableau 6 ont été effectuées avec chaque plastifiant du tableau 2, dans un mélangeur de type à moyenne vitesse.

**Tableau 6: Recette pour pates de PVC - formules type mousse (couches expansées):**

| **Matières Premières** | **Quantité (phr)** |
|---|---|
| SolVin 367NK | 100 |
| Plastifiant | 62 |
| CaCO₃ (15 mm) | 40 |
| Porofor^{®} ADC (50%) + DINP | 6 |
| Baerostab^{®} KK42 (K/Zn) | 2.0 |

La comparaison des données obtenues pour les formulations synthétisées, à chaque fois en modifiant le plastifiant sont résumées dans le tableau 7.

Tableaux 7a et 7b : évaluations et comparaison des propriétés

**7 a- Viscosité et vieillissement de la pate à bas gradient de vitesse (Eta = 1.4 s⁻¹)**

| **Plastifiant** | **t 0** | **t 0+ 24h** |
|---|---|---|
| DEHP | 9,1 | 8,5 |
| DINP | 7,2 | 6,5 |
| DINCH | 7,4 | 5,6 |
| DOTP | 10,6 | 7,5 |
| Nexo E01 | 3,8 | 5,0 |
| Plast Fast | 11,4 | 11,7 |
| MB10/DINP | 2,1 | 2,5 |
| 2088/DINP | 6,9 | 7,3 |

**7 b- densité, couleur, taux d'expansion et qualité cellulaire (thickness: 0.35mm, 2 min at 200°C)**

| **Plastifiant** | **Densité (g/cm3)** | **Vitesse d'expansion** | **Indice Jaune** | **Qualité cell** |
|---|---|---|---|---|
| DEHP | 0,25 | 5,1 | 12,9 | Très bonne |
| DINP | 0,25 | 5,27 | 14,19 | Très bonne |
| DINCH | 0,25 | 5,2 | 15,2 | Très bonne |
| DOTP | 0,26 | 5,2 | 11,25 | Très bonne |
| Nexo E01 | 0,24 | 5,6 | 47,7 | Moyenne |
| Plast Fast | 0,21 | 6,3 | 19,4 | Bonne |
| MB10/DINP | 0,25 | 5,4 | 14 | Très bonne |
| 2088/DINP | 0,25 | 5,2 | 16,2 | Très bonne |

Le tableau 8 récapitule le classement des plastifiants selon les notes attribuées.

**Tableau 8: Récapitulatif des propriétés obtenues avec les plastifiants dans les formulations mousses de PVC**

| **Plastifiant** | **Viscosité** | **Vieillissement** | **Couleur** | **Densité** | **Taux d'Exp** | **Qualité Cell** | **Score** |
|---|---|---|---|---|---|---|---|
| DEHP | -0,5 | 0 | 0,5 | 0 | 0 | 0 | 0 |
| DINP | 0 | 0 | 0 | 0 | 0 | 0 | 0 |
| DINCH | 0 | 0 | 0 | 0 | 0 | 0 | 0 |
| DOTP | -0,75 | 0 | 0,5 | 0 | 0 | 0 | -0,25 |
| Nexo E01 | 1 | 0 | -2,5 | 0,5 | 0,5 | -1 | -1,5 |
| Plast Fast | -0,75 | 0 | -0,75 | 1 | 1,25 | -0,5 | 0,25 |
| MB10/DINP | 1,5 | 0 | 0 | 0 | 0,25 | 0 | 1,75 |
| 2088/DINP | 0 | 0 | 0 | -0,25 | 0 | 0 | -0,25 |

La comparaison générale de ces 2 formulations nous permet d'avoir le résultat de la performance globale des plastifiants. La moyenne du score des couches transparentes et le score des couches mousses nous donne le score global de performance pour chaque plastifiant et nous permet ainsi de les comparer. Le tableau 9 donne le score total des performances évaluées.

**Tableau 9. Score total des performances de chaque plastifiant.**

| **Plastifiant** | **Score couches Transparentes** | **Score couches mousses** | **Score Total** |
|---|---|---|---|
| DEHP | 0,125 | 0 | 0,125 |
| DINP | 0 | 0 | 0 |
| DINCH | -0,75 | 0 | -0,75 |
| DOTP | -0,5 | -0,25 | -0,75 |
| Nexo E01 | 2,875 | -1,5 | 1,38 |
| Plast Fast | 2,75 | 0,25 | 3 |
| MB10/DINP | -0,5 | 1,75 | 1,25 |
| 2088/DINP | -1,8125 | -0,25 | -2,06 |

En moyenne, le « plast fast » présente une bonne performance pour les 2 recettes testées, il a le plus haut score en comparaison avec les autres plastifiants. Ce plastifiant gélifie rapidement, a une viscosité très basse et vieilli très bien. Le débullage efficace est l'une de ces caractéristiques phares. Les mousses préparées avec ce plastifiant ont des densités basses, une grande vitesse d'expansion et d'excellentes qualités.

## Revendications

1. Composition plastifiante comportant une résine et un agent plastifiant qui comporte au moins un diester de formule générale (I) :
RO-OC-A-CO-OR (I)
dans laquelle :
- A est une chaine aliphatique linéaire ou branchée en C4 à C6, et
- R est un alkyle, un cycloalkyle ou un aryle.

2. Composition selon la revendication 1, **caractérisée en ce que** R est un cycloalkyle substitué ou non, de 5 à 6 atomes de carbone, choisi parmi le cyclopentyle ou le cyclohexyle, ou un aryle choisi parmi le benzyle, phényle, et leurs isomères.

3. Composition selon la revendication 1, **caractérisée en ce que** R est un alkyl ramifié ou non, ayant de 4 à 10 d'atome de carbone, en particulier choisi parmi le n-butyle, t-butyle, n-pentyle, n-hexyle, n-heptyle, n-octyle, éthyl-hexyl, iso-octyle, n-nonyle, iso-nonyl, n-décyle, iso-décyle .

4. Composition selon l'une quelconque des revendications précédentes, **caractérisée en ce que** le diester est un dérivé d'un diacide choisi parmi l'acide succinique, l'acide glutarique, l'acide adipique, l'acide éthylsuccinique ou l'acide méthylglutarique.

5. Composition selon l'une quelconque des revendications précédentes, **caractérisée en ce que** l'agent plastifiant est un mélange de :
- 70 à 95% en poids de diester d'acide méthylglutarique,
- 5 à 30% en poids diester d'acide éthylsuccinique, et
- 0 à 10% en poids diester d'acide adipique.

6. Composition selon l'une quelconque des revendications 1 à 5, **caractérisée en ce que** l'agent plastifiant est un mélange de :
- 95 à 100% en poids de diester d'acide méthylglutarique, et
- 0 à 5 % en poids de diester d'acide éthylsuccinique.

7. Composition selon l'une quelconque des revendications précédentes, **caractérisée en ce que** la résine est choisie parmi les polyamides, les bétons, les polyesters, de polyuréthanes, des résines de chlorure de vinyl, des co-polymères éthylène-vinyle d'acétate, des caoutchoucs ou de leurs mélanges.

8. Composition selon l'une quelconque des revendications précédentes, **caractérisée en ce qu'**elle comprend en outre un ou plusieurs composés choisis parmi des stabilisants, les charges, des pigments, des biocides, du charbon noir, des promoteurs d'adhésion, des réducteurs de viscosité, des agents thixotropiques, des agents épaississants, des agents de gonflement, des dispersants, ou autres additifs.

9. Composition selon l'une quelconque des revendications précédentes, **caractérisée en ce que** l'agent plastifiant comprend en outre un ou plusieurs composés choisi parmi le phthalate, l'adipate, le benzoate, le triglycéride, ou autres polymères.

10. Procédé de production d'un diester ou d'un mélange de diesters de formule générale (I) :
RO-OC-A-CO-OR
dans laquelle :
- A est une chaine aliphatique linéaire ou branchée en C4 à C6, et
- R est un alkyle, un cycloalkyle ou un aryle,
**caractérisé en ce qu'**il comporte les étapes suivantes :
- hydrolyse d'un dinitrile ou d'un mélange de dinitriles,
- estérification d'un intermédiaire diacide ou du mélange, en présence d'un alcool,
- décoloration et purification permettant d'obtenir le diester ou le mélange de diesters correspondant.

11. Procédé selon la revendication précédente, **caractérisé en ce que** le dinitrile est choisi parmi l'adiponitrile, le méthyl-glutaronitrile, l'éthyl-succinonitrile, le glutaronitrile, le succinonitrile, ou leur mélange.

12. Procédé selon la revendication 10, **caractérisé en ce qu'**il est produit à partir d'un diacide, ou d'un mélange de diacides, choisi(s) parmi l'acide succinique, l'acide glutarique, l'acide adipique, l'acide éthylsuccinique ou l'acide méthylglutarique.

13. Utilisation de la composition selon l'une quelconque des revendications 1 à 9, pour préparer des articles sélectionnés parmi les jouets, films, feuilles, tubes flexibles, revêtements, câbles, dalles de sol, en particulier à base de vinyle, cuirs synthétiques, adhésifs et encres.

14. Utilisation de la composition selon l'une quelconque des revendications 1 à 9, pour la préparation d'articles par des procédés de mélange à sec ou extrusion.

15. Utilisation de la composition selon l'une quelconque des revendications 1 à 9, pour la préparation de plastisols de type pâte, film ou mousse.
